# EUROPEAN PATENT SPECIFICATION

(11) **EP 1 610 842 B1**
(45) Date of publication and mention of the grant of the patent: **30.09.2009**
(21) Application number: 04726349.6
(22) Date of filing: 07.04.2004
(51) Int. Cl.: A61M 1/16, B01D 29/03, B01D 29/56

(54) **Filter for a medical dialysis cartridge**
Filter für eine Kartusche für medizinische Dialyse
Filtre à Cartouche pour dialyse médicale

(30) Priority: 07.04.2003 SE 0301021; 07.04.2003 US 461659 P
(43) Date of publication of application: 04.01.2006
(73) Proprietor: Gambro Lundia AB, 220 10 Lund (SE)
(72) Inventor: LORENTZON, Jan-Olof, S-380 30 Rockneby (SE); TRYGGVASON, Ragnar, S-240 21 Löddeköpinge (SE)
(74) Representative: Larsson, Eva-Karin
(86) International application number: PCT/SE2004/000557
(87) International publication number: WO 2004/089442

(56) References cited:
- WO-A1-97/29796
- DE-U1- 20 008 860
- SE-B- 467 142
- US-A- 2 847 126
- US-A- 3 209 915
- US-A- 5 566 611
- US-A- 5 637 214

## Description

### BACKGROUND OF THE INVENTION AND PRIOR ART

The present invention refers to a cartridge according to the preamble of claim 1, and the preamble of claim 17.

The present invention also refers to a system for preparing a liquid solution for a medical procedure according to the preamble of claim 33.

The common subject-matter between claims 1, 17 and 33 is a cartridge with a filter with one or more slit-shaped openings.

In a dialysis equipment, it is known to use such a cartridge for the supply of different substances to the dialysis liquid, see EP-B-278 100. The particulate material contained in the cartridge may include various substances to be supplied to the dialysis liquid, such as sodium bicarbonate, sodium chloride and other salts. In use, the dialysis liquid is flowing through the cartridge, wherein the particulate substance is successively dissolved and thus added to the dialysis liquid. Consequently, the general purpose of the filter is to permit the passage of the dialysis liquid together with a dissolved quantity of the substance, but to prevent the passage of any particles of the particulate material. If such particles of the substance are contained in the dialysis liquid, sensible parts of the dialysis equipment, such as pumps, may be damaged.

The filter used today in such a cartridge is made of a fibre material, such as polypropylene, in the form of a woven filter net. Such a filter is flexible, and thus not self-supporting. The filter, therefore, has to be supported by a supporting component, for instance in the form of a support plate. The filter is mounted to the support plate in a position adjacent to the support plate, wherein the filter and support plate form a common unit that is mountable in the cartridge. The manufacture of such a filter unit is relatively complicated and expensive, since the manufacture includes the separate steps of manufacturing the woven filter, manufacturing the support plate and joining the filter and the plate to each other. Moreover, it is difficult to obtain exactly the desired permeability for such a filter. In particular, the permeability frequently is to high, which involves a risk for formation of channels in the particulate material contained in the cartridge.

Another problem which may occur with the cartridges used today is that the particulate material may escape from the cartridge through the inlet and the outlet before the cartridge is actually used, especially via the inlet during the initial priming operation. In order to reduce this risk of escape, a felt pad or any other similar porous member is incorporated in the inlet and the outlet. In particular, during priming from below, via the outlet, the felt pad at the inlet is important. The porous felt pad is normally manufactured in another material than the rest of the cartridge, which is disadvantageous from a recycling point of view. US-5,637,214 describes a filter assembly for a water treatment apparatus. The filter assembly comprises a housing having elongate slots at the outlet end and containing a particulate material forming the filter media. The particulate material is contained in a filter bag having small pores and thus preventing the particulate material from leaving the assembly. The dimensions of the elongate slots are larger than the filter particles.

US-5,566,611 describes an apparatus for separating liquid from fibrous suspensions. No cartridge for housing a particulate material is disclosed.

WO-A-97-29796 describes a cartridge or reservoir for supply of soluble powder in dialysis. The reservoir has a nylon mesh filter.

### SUMMARY OF THE INVENTION

The object of the present invention is to provide an improved cartridge containing a particulate material. In particular, it is aimed at a cartridge, which is inexpensive to manufacture and which demonstrates a uniform and reliable permeability.

This object is achieved by the cartridge initially defined, which is characterised by the features of claim 1.

By such a filter, a uniform flow area of the slit-shaped opening or slit-shaped openings is ensured. Any particles of the particulate material having a minimum size larger than the second extension will be efficiently prevented from passing the filter, and thus from causing any damages to components arranged downstream the filter. Such a filter at the inlet of the cartridge also permits the achievement of a desired pressure drop over the filter. With an appropriate pressure drop a channelling in the particulate material may be avoided. It should also be pointed out that such a filter may be manufactured in an inexpensive manner by means of an injection moulding process.

The feature, that the second extension is also significantly shorter than the length of the slit-shaped opening in the filter direction, permits a certain thickness of the filter ensuring a sufficient strength of the filter. Consequently, the filter is self-supporting and does not need any support plate to be provided adjacent to the filter, which also contributes to reducing the manufacturing costs.

According to an embodiment of the invention, the second extension is equal to or less than 0,12 mm, preferably equal to or less than 0,10 mm or 0,08 mm. Moreover, the second extension may be equal to or more than 0,02 mm, preferably equal to or more than 0,04 mm. According to an advantageous embodiment, the second extension is approximately 0,06 mm.

According to a further embodiment of the invention, the filter is made of a polymer material, including one of polypropylene and polycarbonate.

According to a further embodiment of the invention, the filter includes a filter element, wherein the slit-shaped opening extends through the filter element. Advantageously, the filter includes a plurality of such slit-shaped openings, which extend through the filter element. Such filter element may be easily moulded in one single piece. The first extension of each slit-shaped opening may extend in a radial direction towards a centre point of the filter element, providing a plurality of slit-shaped openings in a star-like configuration.

According to a further embodiment of the invention, the filter element has a shape of a substantially plane disc. Such a shape is easy to manufacture and is suitable for the filter at the inlet and the outlet of the cartridge.

According to another embodiment of the invention, the filter element has a conical shape. Such a conical shape is also easy to manufacture and is suitable for the filter at the inlet and the outlet of the cartridge. This conical shape is particularly suitable for the filter at the inlet of the cartridge. The filter may then be mounted in an attachable cover of the cartridge, wherein the tip of the conical filter element is pointing away from the inner space of the cartridge. Liquid may then flow through the slit-shaped openings and on the conical outer surface of the filter element. Moreover, the filter may include a peripheral support portion connected to the filter element and adapted to abut an inner wall of the cartridge. The peripheral support portion may then advantageously have a peripheral surface and include a plurality of ridges projecting from the peripheral surface and adapted to abut the inner wall of the cartridge so that a thin gap is formed between the peripheral surface and the inner wall, said gap providing a further passage for the liquid. In such a way it may be ensured that a sufficient amount of liquid is always present in the cartridge.

According to a further embodiment of the invention, the slifi-shaped opening has an first end and a second end, wherein the second extension of the slit-shaped opening increases from a minimum value at one of the ends of the slit-shaped opening to a maximum value at the other end of the opening. Such a shape of the slit-shaped opening or openings is advantageous from a manufacturing point of view. If the first end of the openings faces the inner space of the cartridge and thus the particulate material, the particulate material is efficiently prevented from leaving the cartridge. However, even if the second end faces particulate material a proper functioning of the filter is achieved, i.e. particulate material will be efficiently prevented from leaving the cartridge.

According to a further embodiment of the invention, the filter is made through an injection moulding process.

The object is also achieved by the cartridge initially defined, which is characterised by the features of claim 17.

Advantageous embodiments of the cartridge are defined in the dependent claims 18 to 32.

Furthermore, the object is achieved by the system initially defined, which is characterised by the features f claim 33. Advantageous embodiments are defined in claims 34 to 37.

### BRIEF DESCRIPTION OF THE DRAWINGS

The present invention is now to be described more closely by the following description of various embodiments and with reference to the drawings attached.
- Fig 1: discloses schematically a system for preparing a liquid solution for a medical procedure.
- Fig 2: discloses schematically a cartridge according to the present invention.
- Fig 3: discloses schematically a plan view of a first embodiment of a filter to be arranged in the cartridge of Fig 2.
- Fig 4: discloses schematically a sectional view of the filter along the line IV-IV in Fig 3.
- Fig 5: discloses schematically a sectional view of the filter along the line V-V in Fig 3.
- Fig 6: discloses schematically a cartridge having a filter according to a third embodiment of the present invention.
- Fig 7: discloses schematically a perspective view of the filter disclosed in Fig 6.
- Fig 8: discloses schematically another perspective view of the filter disclosed in Fig 6.

### DETAILED DESCRIPTION OF VARIOUS EMBODIMENTS OF THE INVENTION

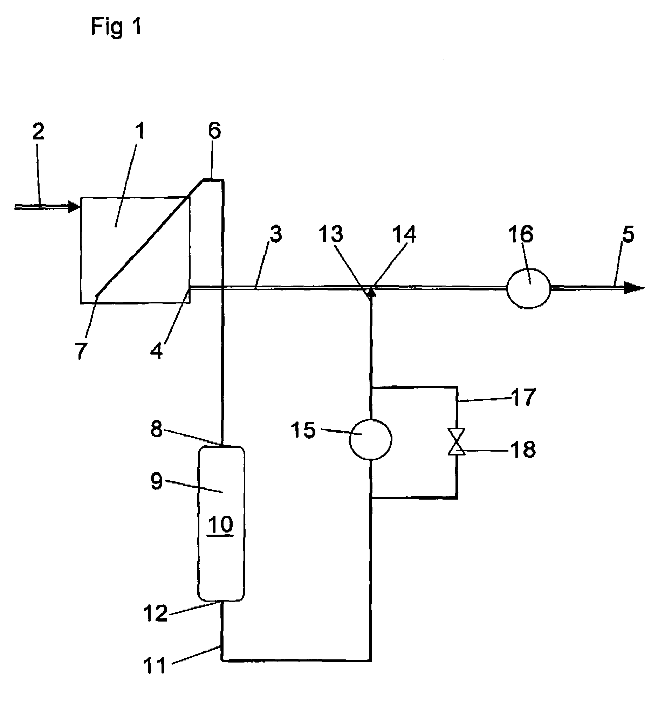

Fig 1 discloses schematically a system for preparing a liquid solution for a medical procedure. In particular, the system is designed for preparing a dialysis liquid solution for the performance of a hemodialysis treatment. The system may also be used for hemodiafiltration and hemofiltration. The system disclosed In Fig 1 may thus form a part of a dialysis equipment.

The system includes a source 1 containing a liquid, and in particular a dialysis liquid. The source 1 may be supplied with the liquid via an inlet conduit 2. A first liquid conduit 3 has a first end 4 communicating with the source 1 to withdraw the dialysis liquid into the first liquid conduit 3. The first liquid conduit 3 also has a second end 5 for delivering a dialysis liquid solution to a receiver (not disclosed) such as a dialysis equipment. A second liquid conduit 6 has a first end 7 communicating with the source 1 and a second end 8 communicating with the inlet of a cartridge 9 for the introduction of the liquid from the source 1 into an inner space 10 of the cartridge 9 to produce a concentrate liquid solution. A third liquid conduit 11 has a first end 12 communicating with the outlet of the cartridge 9 and a second end 14 communicating with the first liquid conduit 3 at a mixing point 13 intermediate said first end 4 and said second end 5.

The third liquid conduit 11 is thus arranged to withdraw said concentrate liquid solution from the cartridge 9 Into the first liquid conduit 3 to be mixed with the liquid conducted through the first liquid conduit 3 from the source 1 in order to produce a liquid solution for delivery to said receiver. The concentrate liquid solution is transported through the third liquid conduit 11 by means of a pump 15. The liquid is transported from the source 1 to said receiver through the first liquid conduit 3 by means of a pump 16. A control loop 17 including a valve 18 may be arranged to control the quantity of concentrate liquid solution to be delivered to the liquid of the first liquid conduit 3.

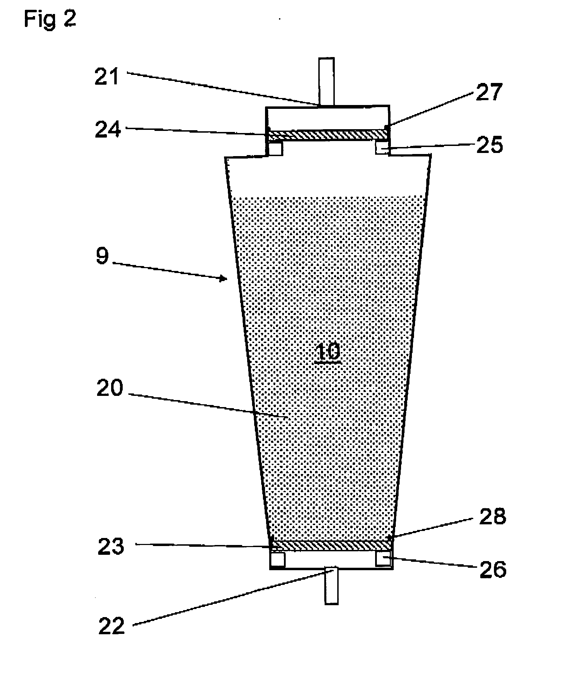

The cartridge 9 is explained below with reference to Fig 2. In the embodiment disclosed, it is referred to the production of a dialysis liquid solution to which sodium bicarbonate has been added. It is to be noted that also other substances than sodium bicarbonate can be added in a similar manner to the dialysis liquid, for Instance sodium chloride and other salts. It is also to be noted, that the system, the cartridge and the components included therein can be used also for producing other liquid solutions than a dialysis liquid.

The cartridge 9 is arranged to contain a particulate material 20 in the inner space 10. In the embodiment disclosed the particulate material 20 is a sodium bicarbonate powder. The cartridge 9 has an inlet 21 which is arranged to permit the introduction of a liquid into the inner space 10. In the embodiment disclosed the inlet 21 is connected to and communicates with the second liquid conduit 6. The cartridge 9 also has an outlet 22 arranged to permit the discharge of liquid from the inner space 10. In the embodiment disclosed, the outlet 22 is connected to and communicates with the third liquid conduit 11 for discharging the concentrate liquid solution.

A first filter 23 is arranged at the outlet 22 to permit passage of said liquid through the filter 23, but to prevent the passage of the particulate material 20 through the filter 23. The filter 23 defines a filter direction x, and thus permits the liquid to pass through the filter 23 in the filter direction x. The cartridge 9 also includes a second filter 24 arranged at the Inlet 21 to permit passage of the liquid through the filter 24, but to prevent passage of the particulate material 20 through the filter 24. Also the second filter 24 defines a filter direction x and permits the liquid to pass through the filter 24 in the filter direction x. It is to be noted that according to this invention such a filter 23 is provided at the outlet 22. The provision of the second filter 24 at the inlet 21 is preferable, but not mandatory.

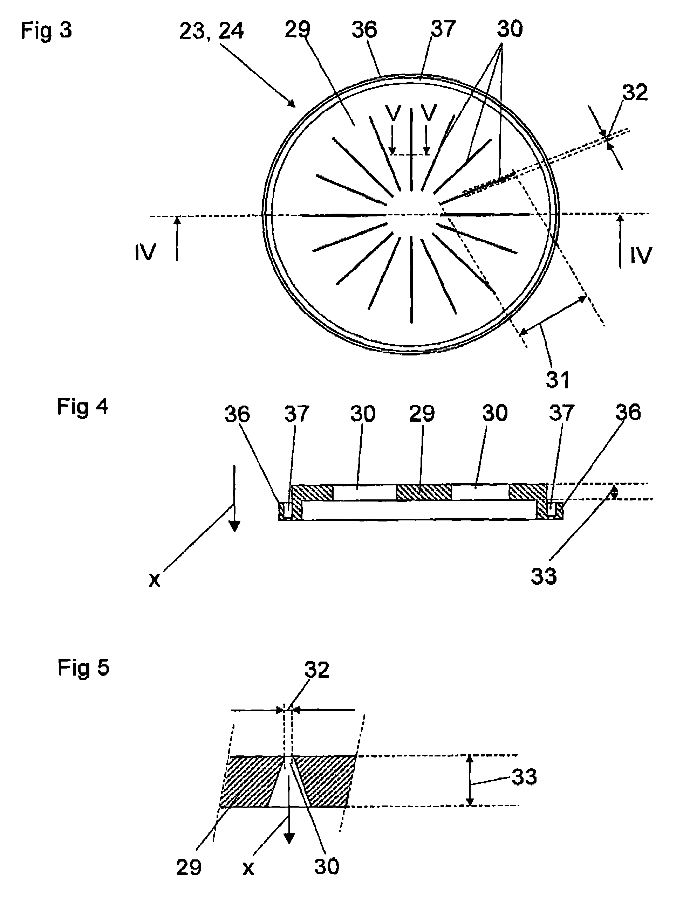

The filters 23 and 24 disclosed in Fig 3 rest on a respective support member in the form of a shoulder 25, 26 extending around the Inner periphery of the inner space 10 In the proximity of the inlet 21 and the outlet 22, respectively. Furthermore, a respective smaller shoulder 27 and 28 extends around the Inner periphery of the inner space 10 above the filter 24 and 23, respectively, in order to permit the locking of the filters 23, 24 at their respective position on the shoulders 25, 26.

Preferably, the filters 23 and 24 are identical to each other. Two different embodiments of the filters 23, 24 are explained below with reference to Figs 3-7.

Figs 3-5 disclose a first embodiment of the filter 23, 24. The filter 23, 24 has a filter element with the shape of a substantially circular and substantially plane disc 29 having a main extension plane. It is to be noted, that the thickness of the disc 29 is exaggerated in the figures in order to simplify the explanation of the filter construction. The filter 23, 24 includes a number of slit-shaped openings 30. Each such opening 30 has a first extension, indicated by the line 31 in Fig 3 for one of the openings 30. Each opening 30 also has a second extension, indicated by the line 32 in Figs 3 and 5. The second extension 32 is substantially perpendicular to the first extension 31. Both the second extension 32 and the first extension 31 are substantially perpendicular to the filter direction x.

In accordance with the present invention, the second extension 32 is significantly shorter than the first extension 31. Moreover, the second extension 32 is significantly shorter than the length 33 of the slit-shaped opening 30 in the filter direction x. For instance, the length 33 may be 10 - 100 times the second extension 32. In particular, the second extension 32 is equal to or less than 0,12 mm, preferably equal to or less than 0,10 mm or even 0,08 mm. Moreover, the second extension 32 is equal to or more than 0,02 mm, preferably equal to or more than 0,04 mm. According to a preferred embodiment, the second extension 32 is approximately 0,06 mm.

The slit-shaped openings 30, which extend through the disc 29, are, in the embodiment disclosed in Figs 3-5, provided to extend in a radial direction with regard to the first extension 31 and a centre point of the disc 29. However, the slit-shaped openings 30 may also be arranged to extend in other directions, for instance the disc 29 may include a plurality of slit-shaped openings 30 extending in parallel to each other. The disc 29 is made in a polymer material, for instance polycarbonate or polypropylene. The disc 29 may be manufactured through an Injection moulding process by injecting said polymer material into a mould cavity having the shape of the disc 29. Each slit-shaped opening 30 has a first end and a second end. In Fig 5 the first end is an upstream end and the second end a downstream end with respect to the filter direction x. As appears from Fig 5, the second extension 32 of the slit-shaped opening 32 increases in the filter direction x from a minimum value at the upstream end of the slit-shaped opening 30 to a maximum value at the downstream end of the slit-shaped opening 30. Such a shape of the slit-shaped opening facilitates the injection moulding of the disc 29. It is to be noted here that the slit-shaped openings 30 of the filter 23, 24 may also be arranged in an opposite manner with respect to the filter direction x, depending on the particular circumstances. For instance it could in same cases be advantageous if the thinnest end of the slit-shaped openings 30 faces the particulate material although this is not necessary for a proper functioning of the filter 23, 24.

The filter 23, 24 includes a circumferential flexible edge portion 36 formed as a part of the disc 29. The edge portion 36 is flexible in an radial inward direction and formed by the provision of a circumferential groove 37 extending radially inside the edge portion 36. Consequently, the flexible edge portion 36 may be bent radially inwardly when the filter 23, 24 is introduced at its position on the shoulder 25, 26 in order to pass the shoulder 27, 28. After having passed the shoulder 27, 28, the flexible edge portion 36 may flex back to the outer position disclosed in Fig 4.

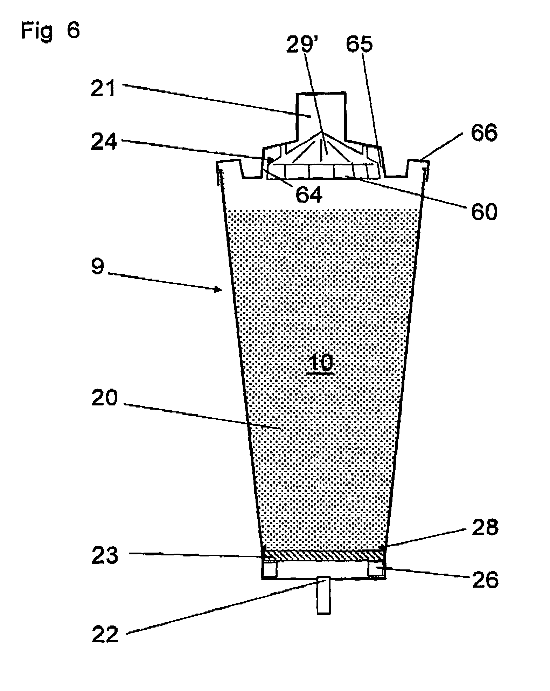

Fig 6 discloses the cartridge 9 with a second filter 24 according to a third embodiment. The second filter 24 is arranged at the inlet 21 to permit passage of the liquid through the second filter 24, but to prevent passage of the particulate material 20 through the second filter 24. It is pointed out that the size of the filter 24 in relation to cartridge may be different than disclosed in Fig 6. Advantageously, the second filter 24 is significantly smaller in relation to the cartridge 9 than disclosed in Fig 6.

Also in this case the second filter 24 defines a filter direction x and permits the liquid to pass through the filter 24 in the filter direction x. The second filter 24 according to the third embodiment is disclosed more closely in Figs 9 and 10. The second filter 24 includes a filter element 29' that has a conical shape. The filter element 29' is relatively thin and has a thickness of material corresponding to the thickness of material of the disc 29 shown in the first embodiment. The filter element 29' thus has a conical inner surface facing the inner space of the cartridge 9 and an opposite conical outer surface. A plurality of slit-shaped openings 30 extend through the filter element 29'. The first extension 31 of each slit-shaped opening 30 extends in a radial direction towards a centre point of the filter element 29', whereas the second extension 32 extends in a tangential direction. The dimensions of the first extension 31 and the second extension 32 are substantially the same as in the first embodiment. Since the thickness of material is substantially the same as in the first embodiment the length 33, see Fig 5 is substantially the same as in the first embodiment.

The second filter 24 according to the third embodiment also includes a peripheral support portion 60 connected to a lower end portion of the filter element 29'. The peripheral support portion 60 has a peripheral surface 61 and includes a plurality of longitudinal ridges 62 projecting from the peripheral surface 61 and extending in a longitudinal direction, nearly in parallel to each other. The peripheral surface 61 is nearly cylindrical or slightly conical so that the peripheral surface is somewhat tapering, with a small cone angle, towards the filter element 29'. The peripheral support portion 60 abuts an inner wall 64 of the cartridge 9. More precisely, the longitudinal ridges 62 abut the inner wall 64 of the cartridge 9 in such a way that a thin gap 65 is formed between the peripheral surface 61 and the inner wall 64. The gap 65, which is divided into a plurality of gap portions by the ridges 62, provides a further passage for the liquid. The height of the ridges 62 is selected in such a way that the width of the gap 65 is substantially the same as the length of the second extension 32. In the embodiment disclosed in Figs 6, the inner wall 64 is formed by a cover 66, which is attachable to an upper end of the cartridge 9 for closing the cartridge 9 when it has been filled with the particulate material. The inner wall 64 has substantially the same cone angel as the peripheral surface 61. The second filter 24 of the third embodiment is attached to the cover 66 by being pressed against the inner wall 64. The second filter 24 of the third embodiment also includes a number of support posts 67, in the embodiment disclosed four posts 67, which are abutting an upper wall adjoining the slightly conical inner wall 64 for defining the position of the filter with respect to the cartridge 9, or more precisely the cover 66. Furthermore, the second filter 24 of the third embodiment includes a number of supporting wings, in the embodiment disclosed four wings 68, which are arranged on an inner peripheral surface being opposite to the peripheral surface 61 of the peripheral support 60, and on the conical inner surface of the filter element 29'. The wings 68 extend in a radial direction. The cover 66 and the second filter 24 according to the third embodiment are designed in such a manner that assemblies of the cover 66 and the second filter 24 may be stacked onto each other during manufacturing and transport. Thereby the pipe-shaped inlet 21 of the cover is dimensioned to extend into the second filter of an adjacent assembly so that the height of the package of assemblies is minimised.

The slit-shaped openings 30 of the filter element 29' of the second filter 24 according to the third embodiment also has a first end and a second end. In the embodiment disclosed the first end is an upstream end and the second end a downstream end with respect to the filter direction x. Moreover, in the embodiment disclosed, the second extension 32 of the slit-shaped opening 30 decreases in the filter direction x from a maximum value at the first, upstream end of the slit-shaped opening 30 to a minimum value at the second, downstream end of the opening 30. Also the second filter 23 and its filter element 29' according to the third embodiment is made through an injection moulding process.

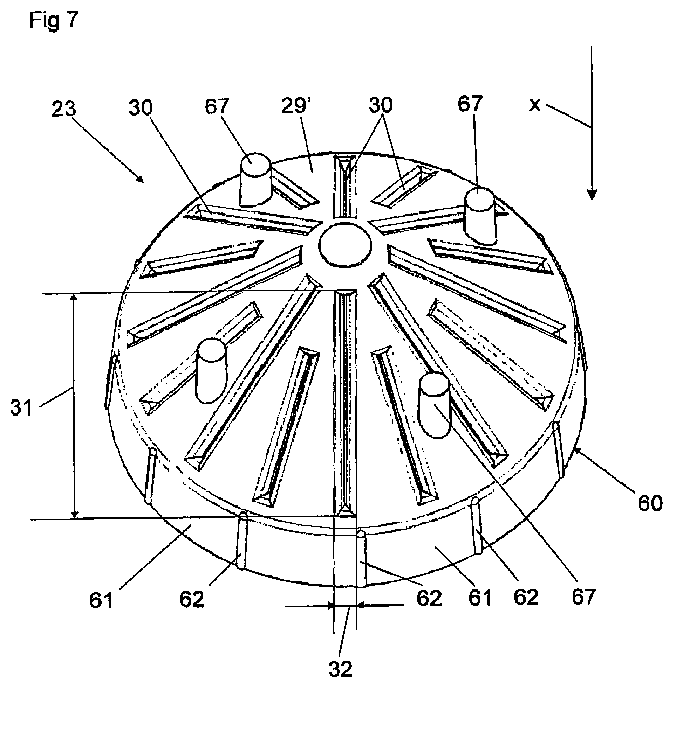

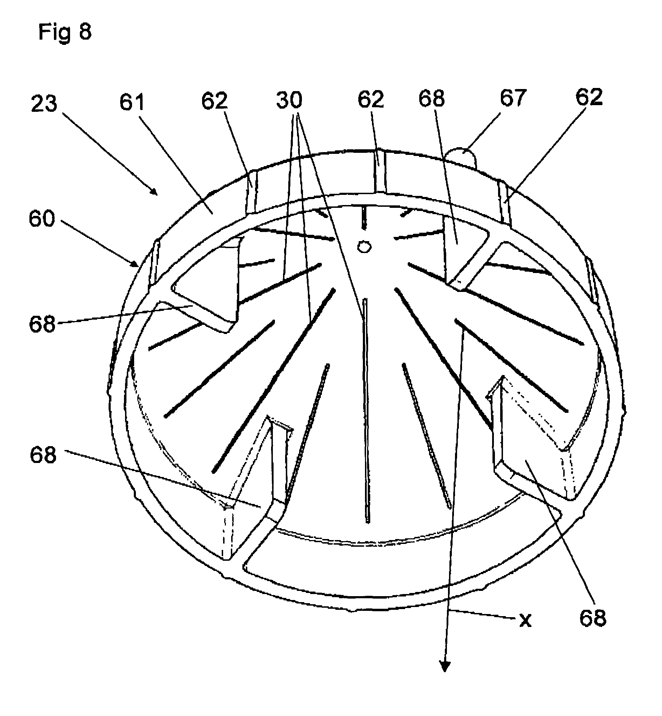

It is to be noted that the filter according to the third embodiment disclosed in Figs 7 and 8 may also be used as the first filter at the outlet 22 of the cartridge 9. In this case the filter element 29' may be arranged with the tip of the cone pointing away from the cartridge, i.e. the filter is turned 180° with respect to the filter direction x. Also when provided at the outlet 22, the height of the ridges 62 is selected in such a way that the width of the gap is substantially the same as the length of the second extension 32. It is also possible, in principle, to arrange one or both of the filters 23, 24 with the cone pointing inwardly towards the particulate material.

It is also to be noted that the cartridge may be used in any direction so that the outlet 22 becomes the inlet 21 and the inlet 21 becomes the outlet 22.

The present invention is not limited to the embodiments disclosed but may be varied and modified within the scope of the following claims. In the embodiment disclosed in Figs 3-5 and 6-8, the first extension 31 is substantially straight. It is to be noted, however, that the first extension 31 as an alternative may be curved.

## Claims

1. A cartridge containing a particulate material (20), wherein the cartridge includes:
an inner space (10) for housing the particulate material;
an inlet (21) arranged to permit the introduction of a liquid into the inner space (10);
an outlet (22) arranged to permit the discharge of liquid from the inner space (10); and
at least a first filter (23) arranged at the outlet (22) and to permit passage of the liquid through the filter, but to prevent passage of the particulate material (20) through the filter, wherein the filter permits the liquid to pass through the filter in a filter direction (x),
wherein the filter (23) includes at least one slit-shaped opening (30), which has a first extension (31) and a second extension (32) being substantially perpendicular to the filter direction (x) and to the first extension, wherein the second extension (32) is significantly shorter than the first extension (31),
**characterised in that** the second extension (32) is significantly shorter than the length of the slit-shaped opening in the filter direction (x).

2. A cartridge according to claim 1, wherein the cartridge includes a second filter (24) arranged at the inlet (21) and to permit passage of the liquid through the filter (24), but to prevent passage of the particulate material (20) through the filter (24), wherein the second filter permits the liquid to pass through the filter (24) in a filter direction (x), **characterised in that** the second filter includes at least one slit-shaped opening, which has a first extension (31) and a second extension (32) being substantially perpendicular to the filter direction (x) and to the first extension (31), wherein the second extension (32) is significantly shorter than the first extension (31).

3. A cartridge according to any one of claims 1 and 2, **characterised in that** the second extension (32) is equal to or less than 0,12 mm.

4. A cartridge according to any one of claims 1 to 3, **characterised in that** the second extension (32) is equal to or less than 0,10 mm or 0,08 mm.

5. A cartridge according to any one of claims 1 to 4, **characterised in that** the second extension (32) is equal to or more than 0,02 mm.

6. A cartridge according to any one of claims 1 to 5, **characterised in that** the second extension (32) is equal to or more than 0,04 mm.

7. A cartridge according to any one of claims 1 to 6, **characterised in that** the second extension (32) is approximately 0,06 mm.

8. A cartridge according to any one of claims 1 to 7, **characterised in that** the filter (23, 24) is made of a polymer material, including one of polypropylene and polycarbonate.

9. A cartridge according to any one of claims 1 to 8, **characterised in that** the first extension (31) is substantially perpendicular to the filter direction (x).

10. A cartridge according to any one of claims 1 to 9, **characterised in that** the filter (23, 24) includes a filter element (29, 29'), wherein the slit-shaped opening (30) extends through the filter element.

11. A cartridge according to claim 10, **characterised in that** the filter (23, 24) includes a plurality of slit-shaped openings (30), which extend through the filter element (29, 29').

12. A cartridge according to claim 11, **characterised in that** the first extension (31) of each slit-shaped opening (30) extends in a radial direction towards a centre point of the filter element (29, 29').

13. A cartridge according to any one of claims 10 to 12, **characterised in that** the filter element (29) has a shape of a substantially plane disc.

14. A cartridge according to claim 2 and 10, **characterised in that** the filter element (29') of the second filter (24) has a conical shape.

15. A cartridge according to any one of claims 10 to 14, **characterised in that** the slit-shaped opening (30) of the filter element (29, 29') has a first end and a second end, wherein the second extension (32) of the slit-shaped opening increases from a minimum value at one of the ends of the slit-shaped opening to a maximum value at the other end of the opening (30).

16. A cartridge according to any one of claims 1 to 15, **characterised in that** the filter (23, 24) is made through an injection moulding process.

17. A cartridge arranged to contain a particulate material (20), wherein the cartridge includes:
an inner space (10) for housing the particulate material;
an inlet (21) arranged to permit the introduction of a liquid into the inner space (10);
an outlet (22) arranged to permit the discharge of liquid from the inner space (10); and
at least a second filter (24) arranged at the inlet (21) and to permit passage of the liquid through the filter (24), but to prevent passage of the particulate material (20) through the filter (24), wherein the second filter permits the liquid to pass through the filter (24) in a filter direction (x),
wherein the second filter includes at least one slit-shaped opening, which has a first extension (31) and a second extension (32) being substantially perpendicular to the filter direction (x) and to the first extension (31), wherein the second extension (32) is significantly shorter than the first extension (31),
**characterised in that** the second extension (32) is significantly shorter than the length of the slit-shaped opening in the filter direction (x).

18. A cartridge according to claim 17, **characterised in that** the second extension (32) is equal to or less than 0,12 mm.

19. A cartridge according to any one of claims 17 and 18, **characterised in that** the second extension (32) is equal to or less than 0,10 mm or 0,08 mm.

20. A cartridge according to any one of claims 17 to 19, **characterised in that** the second extension (32) is equal to or more than 0,02 mm.

21. A cartridge according to any one of claims 17 to 20, **characterised in that** the second extension (32) is equal to or more than 0,04 mm.

22. A cartridge according to any one of claims 17 to 21, **characterised in that** the second extension (32) is approximately 0,06 mm.

23. A cartridge according to any one of claims 17 to 22, **characterised in that** the filter (23, 24) is made of a polymer material, including one of polypropylene and polycarbonate.

24. A cartridge according to any one of claims 17 to 23, **characterised in that** the first extension (31) is substantially perpendicular to the filter direction (x).

25. A cartridge according to any one of claims 17 to 24, **characterised in that** the filter (24) includes a filter element (29'), wherein the slit-shaped opening (30) extends through the filter element.

26. A cartridge according to claim 25, **characterised in that** the filter (23, 24) includes a plurality of slit-shaped openings (30), which extend through the filter element (29').

27. A cartridge according to claim 26, **characterised in that** the first extension (31) of each slit-shaped opening (30) extends in a radial direction towards a centre point of the filter element (29').

28. A cartridge according to any one of claims 25 to 27, **characterised in that** the filter element (29) has a conical shape.

29. A cartridge according to any one of claims 25 to 28, **characterised in that** the filter (24) includes a peripheral support portion connected to the filter element (29') and abutting an inner wall of the cartridge.

30. A cartridge according to claim 29, **characterised in that** the peripheral support portion has a peripheral surface and includes a plurality of ridges projecting from the peripheral surface and abutting the inner wall of the cartridge, wherein a thin gap is formed between the peripheral surface and the inner wall, said gap providing a further passage for the liquid.

31. A cartridge according to any on of claims 26 to 30, **characterised in that** the slit-shaped opening (30) of the filter element (29') has a first end and a second end, wherein the second extension (32) of the slit-shaped opening decreases from a maximum value at the first end of the slit-shaped opening to a minimum value at the second end of the opening (30).

32. A cartridge according to any one of claims 17 to 31, **characterised in that** the filter (23, 24) is made through an injection moulding process.

33. A system for preparing a liquid solution for a medical procedure; the system including:
a cartridge containing a particulate material in an inner space thereof and including an inlet (21) and an outlet (22);
a first liquid conduit (3) having a first end (4) communicating with a source (1) of liquid to withdraw the liquid into the first liquid conduit (3) and a second end;
a second liquid conduit (6) having a first end (7) communicating with a source (1) of liquid and a second end (8) communicating with the inlet of the cartridge (9) for introducing the liquid into the inner space (10) to produce a concentrate liquid solution containing at least a part of the particulate material dissolved in the liquid;
a third liquid conduit (11) communicating with the outlet of the cartridge and with a mixing point (13) in the first liquid conduit (3) intermediate said first and second ends (4, 5) for conducting said concentrate liquid solution from the cartridge (9) into said first liquid conduit to be mixed with the liquid being conducted through the first liquid conduit to thereby produce said liquid solution in the first liquid conduit for delivery to said second end of the first liquid conduit; and
at least one filter (23) arranged at the outlet (22) and to permit passage of the liquid through the filter, but to prevent passage of the particulate material through the filter, wherein the filter (23) permits the liquid to pass through the filter in a filter direction (x),
wherein the filter includes at least one slit-shaped opening (30), which has a first extension (31) and a second extension (32) being substantially perpendicular to the filter direction (x) and to the first extension (31), wherein the second extension (32) is significantly shorter than the first extension (31), and wherein
the second extension (32) is significantly shorter than the length of the slit-shaped opening in the filter direction (x)

34. A system according to claim 33, wherein the cartridge includes a second filter (24) arranged at the inlet (21) and to permit passage of the liquid through the filter (24), but to prevent passage of the particulate material (20) through the filter (24), wherein the second filter permits the liquid to pass through the filter (24) in a filter direction (x), **characterised in that** the second filter includes at least one slit-shaped opening, which has a first extension (31) and a second extension (32) being substantially perpendicular to the filter direction (x) and to the first extension (31), wherein the second extension (32) is significantly shorter than the first extension (31).

35. A system according to any one of claims 33 and 34, **characterised in that** the filter includes the features of any one of claims 2 to 16.

36. A system according to any one of claims 33 to 35, wherein the liquid is a dialysis liquid.

37. A system according to any one of claims 33 to 36, wherein the particulate material includes bicarbonate and/or sodium chloride.

## Patentansprüche

1. Kartusche mit einem partikelförmigen Material (20), wobei die Kartusche umfasst:
einen Innenraum (10) zur Beherbergung des partikelförmigen Materials;
einen Einlass (21), der angeordnet ist, um das Einlassen einer Flüssigkeit in den Innenraum (10) zu ermöglichen;
einen Auslass (22), der angeordnet ist, um das Ablassen der Flüssigkeit aus dem Innenraum (10) zu ermöglichen; und
zumindest einen ersten Filter (23), der an dem Auslass (22) angeordnet ist, um einen Durchgang der Flüssigkeit durch den Filter zu ermöglichen, aber einen Durchgang des partikelförmigen Materials (20) durch den Filter zu verhindern, wobei der Filter der Flüssigkeit erlaubt, durch den Filter in eine Filterrichtung (x) zu fließen,
wobei der Filter (23) zumindest eine schlitzförmige Öffnung (30) einschließt, welche eine erste Verlängerung (31) und eine zweite Verlängerung (32) aufweist, die im Wesentlichen senkrecht zu der Filterrichtung (x) und der ersten Verlängerung ist, wobei die zweite Verlängerung (32) deutlich kürzer als die erste Verlängerung (31) ist,
**dadurch gekennzeichnet, dass** die zweite Verlängerung (32) deutlich kürzer als die Länge der schlitzförmigen Öffnung in der Filterrichtung (x) ist.

2. Kartusche nach Anspruch 1, wobei die Kartusche einen zweiten Filter (24) einschleißt, der an dem Einlass (21) angeordnet ist und einen Durchgang der Flüssigkeit durch den Filter (24) erlaubt, aber den Durchgang des partikelförmigen Materials (20) durch den Filter (24) verhindert, wobei der zweite Filter der Flüssigkeit erlaubt, durch den Filter (24) in einer Filterrichtung (x) hindurchzugehen, **dadurch gekennzeichnet, dass** der zweite Filter zumindest eine schlitzförmige Öffnung einschließt, welche eine erste Verlängerung (31) und eine zweite Verlängerung (32) aufweist, die im Wesentlichen senkrecht zu der Filterrichtung (x) und zu der ersten Verlängerung (31) ist, wobei die zweite Verlängerung (32) deutlich kürzer als die erste Verlängerung (31) ist.

3. Kartusche nach irgendeinem der Ansprüche 1 und 2, **dadurch gekennzeichnet, dass** die zweite Verlängerung (32) gleich oder kürzer als 0,12 mm ist.

4. Kartusche nach irgendeinem der Ansprüche 1 bis 3, **dadurch gekennzeichnet, dass** die zweite Verlängerung (32) gleich oder kürzer als 0,10 mm oder 0,08 mm ist.

5. Kartusche nach irgendeinem der Ansprüche 1 bis 4, **dadurch gekennzeichnet, dass** die zweite Verlängerung (32) gleich oder größer als 0,02 mm ist.

6. Kartusche nach irgendeinem der Ansprüche 1 bis 4, **dadurch gekennzeichnet, dass** die zweite Verlängerung (32) gleich oder größer als 0,04 mm ist.

7. Kartusche nach irgendeinem der Ansprüche 1 bis 6, **dadurch gekennzeichnet, dass** die zweite Verlängerung (32) ungefähr 0,06 mm lang ist.

8. Kartusche nach irgendeinem der Ansprüche 1 bis 7, **dadurch gekennzeichnet, dass** der Filter (23, 24) aus einem Polymermaterial hergestellt ist, einschließlich einem von Polypropylen und Polycarbonat.

9. Kartusche nach irgendeinem der Ansprüche 1 bis 8, **dadurch gekennzeichnet, dass** die erste Verlängerung (31) im Wesentlichen senkrecht zu der Filterrichtung (x) ist.

10. Kartusche nach irgendeinem der Ansprüche 1 bis 9, **dadurch gekennzeichnet, dass** der Filter (23, 24) ein Filterelement (29, 29') einschließt, wobei sich die schlitzförmige Öffnung (30) durch das Filterelement erstreckt.

11. Kartusche nach Anspruch 10, **dadurch gekennzeichnet, dass** der Filter (23, 24) eine Vielzahl schlitzförmiger Öffnungen (30) einschließt, welche sich durch das Filterelement (29, 29') erstrecken.

12. Kartusche nach Anspruch 11, **dadurch gekennzeichnet, dass** sich die erste Verlängerung (31) jeder schlitzförmigen Öffnung (30) in einer radialen Richtung in Richtung eines Mittelpunkts des Filterelements (29, 29') erstreckt.

13. Kartusche nach irgendeinem der Ansprüche 10 bis 12, **dadurch gekennzeichnet, dass** das Filterelement (29) eine Form einer im Wesentlichen ebenen Scheibe aufweist.

14. Kartusche nach Anspruch 2 und 10, **dadurch gekennzeichnet, dass** das Filterelement (29') des zweiten Filters (24) eine konische Form aufweist.

15. Kartusche nach irgendeinem der Ansprüche 10 bis 14, **dadurch gekennzeichnet, dass** die schlitzförmige Öffnung (30) des Filterelements (29, 29') ein erstes Ende und ein zweites Ende aufweist, wobei sich die zweite Verlängerung (32) der schlitzförmigen Öffnung von einem Minimalwert an einem der Enden der schlitzförmigen Öffnung auf einen Maximalwert an dem anderen Ende der Öffnung (30) vergrößert.

16. Kartusche nach irgendeinem der Ansprüche 1 bis 15, **dadurch gekennzeichnet, dass** der Filter (23, 24) durch ein Spritzgussverfahren hergestellt ist.

17. Kartusche, die angeordnet ist, um ein partikelförmiges Material (20) zu beinhalten, wobei die Kartusche umfasst:
einen Innenraum (10) zur Aufnahme des partikelförmigen Materials;
einen Einlass (21), der angeordnet ist, um das Einfließen einer Flüssigkeit in den Innenraum (10) zu ermöglichen;
einen Auslass (22), der angeordnet ist, um das Ablassen der Flüssigkeit aus dem Innenraum (10) zu ermöglichen; und
zumindest einen zweiten Filter (24), der an dem Einlass (21) angeordnet ist, um den Durchgang der Flüssigkeit durch den Filter (24) zu ermöglichen, aber den Durchgang des partikelförmigen Materials (20) durch den Filter (24) zu verhindern, wobei der zweite Filter der Flüssigkeit das Hindurchgehen durch den Filter (24) in einer Filterrichtung (x) ermöglicht,
wobei der zweite Filter zumindest eine schlitzförmige Öffnung einschließt, welche eine erste Verlängerung (31) und eine zweite Verlängerung (32) aufweist, die im Wesentlichen senkrecht zu der Filterrichtung (x) und der ersten Verlängerung (31) ist, wobei die zweite Verlängerung (32) deutlich kürzer als die erste Verlängerung (31) ist,
**dadurch gekennzeichnet, dass** die zweite Verlängerung (32) wesentlich kürzer als die Länge der schlitzförmigen Öffnung in der Filterrichtung (x) ist.

18. Kartusche nach Anspruch 17, **dadurch gekennzeichnet, dass** die zweite Verlängerung (32) gleich oder kürzer als 0,12 mm ist.

19. Kartusche nach irgendeinem der Ansprüche 17 und 18, **dadurch gekennzeichnet, dass** die zweite Verlängerung (32) gleich oder kürzer als 0,10 mm oder 0,08 mm ist.

20. Kartusche nach irgendeinem der Ansprüche 17 bis 19, **dadurch gekennzeichnet, dass** die zweite Verlängerung (32) gleich oder größer als 0,02 mm ist.

21. Kartusche nach irgendeinem der Ansprüche 17 bis 20, **dadurch gekennzeichnet, dass** die zweite Verlängerung (32) gleich oder größer als 0,04 mm ist.

22. Kartusche nach irgendeinem der Ansprüche 17 bis 21, **dadurch gekennzeichnet, dass** die zweite Verlängerung (32) ungefähr 0,06 mm beträgt.

23. Kartusche nach irgendeinem der Ansprüche 17 bis 22, **dadurch gekennzeichnet, dass** der Filter (23, 24) aus einem Polymermaterial besteht, einschließlich einem von Polypropylen und Polycarbonat.

24. Kartusche nach irgendeinem der Ansprüche 17 bis 23, **dadurch gekennzeichnet, dass** die erste Verlängerung (31) im Wesentlichen senkrecht zu der Filterrichtung (x) steht.

25. Kartusche nach irgendeinem der Ansprüche 17 bis 24, **dadurch gekennzeichnet, dass** der Filter (24) ein Filterelement (29') einschließt, wobei sich die schlitzförmige Öffnung (30) durch das Filterelement erstreckt.

26. Kartusche nach Anspruch 25, **dadurch gekennzeichnet, dass** der Filter (23, 24) eine Vielzahl schlitzförmiger Öffnungen (30) einschließt, welche sich durch das Filterelement (29') erstrecken.

27. Kartusche nach Anspruch 26, **dadurch gekennzeichnet, dass** sich die erste Verlängerung (31) jeder schlitzförmigen Öffnung (30) in einer radialen Richtung in Richtung eines Mittelpunkts des Filterelements (29') erstreckt.

28. Kartusche nach irgendeinem der Ansprüche 25 bis 27, **dadurch gekennzeichnet, dass** das Filterelement (29) eine konische Form aufweist.

29. Kartusche nach irgendeinem der Ansprüche 25 bis 28, **dadurch gekennzeichnet, dass** der Filter (24) einen Umfangsunterstützungsabschnitt einschließt, der mit dem Filterelement (29') verbunden ist, und an der Innenwand der Kartusche anstößt.

30. Kartusche nach Anspruch 29, **dadurch gekennzeichnet, dass** der Umfangsunterstützungsabschnitt eine Umfangsoberfläche aufweist und eine Vielzahl von Rippen einschließt, die von der Umfangsoberfläche hervorstehen und an die Innenwand der Kartusche anstoßen, wobei ein kleiner Spalt zwischen der Umfangsoberfläche und der Innenwand gebildet ist, wobei dieser Spalt einen weiteren Durchgang für die Flüssigkeit bereitstellt.

31. Kartusche nach irgendeinem der Ansprüche 26 bis 30, **dadurch gekennzeichnet, dass** die schlitzförmige Öffnung (30) des Filterelements (29') ein erstes Ende und ein zweites Ende aufweist, wobei die zweite Verlängerung (32) der schlitzförmigen Öffnung von einem Maximalwert an dem ersten Ende der schlitzförmigen Öffnung auf einen Minimalwert an dem zweiten Ende der Öffnung (30) abnimmt.

32. Kartusche nach irgendeinem der Ansprüche 17 bis 31, **dadurch gekennzeichnet, dass** der Filter (23, 24) durch ein Spritzgussverfahren hergestellt ist.

33. System zur Vorbereitung einer flüssigen Lösung für ein medizinisches Verfahren, wobei das System umfasst:
eine Kartusche, die ein partikelförmiges Material in einem Innenraum davon beinhaltet, und einen Einlass (21) und einen Auslass (22) umfasst;
eine erste Flüssigkeitsleitung (3) mit einem ersten Ende (4) in Kommunikation mit einer Flüssigkeitsquelle (1), um die Flüssigkeit in die erste Flüssigkeitsleitung (3) und ein zweites Ende zu ziehen;
eine zweite Flüssigkeitsleitung (6) mit einem ersten Ende (7) in Kommunikation mit einer Flüssigkeitsquelle (1) und einem zweiten Ende (8) in Kommunikation mit dem Einlass der Kartusche (9) zum Einlassen der Flüssigkeit in den Innenraum (10), um eine konzentrierte Flüssigkeitslösung zu erzeugen, die zumindest einen Teil des partikelförmigen Materials gelöst in der Flüssigkeit beinhaltet;
und eine dritte Flüssigkeitsleitung (11) in Kommunikation mit dem Auslass der Kartusche und mit einem Mischungspunkt (13) in der ersten Flüssigkeitsleitung (3) zwischen den ersten und zweiten Enden (4, 5) zum Leiten der konzentrierten Flüssigkeitslösung von der Kartusche (9) in die erste Flüssigkeitsleitung zum Mischen mit der Flüssigkeit, die durch die erste Flüssigkeitsleitung geleitet wird, um dabei die flüssige Lösung in der ersten Flüssigkeitsleitung zum Transport zu dem zweiten Ende der ersten Flüssigkeitsleitung zu erzeugen; und
zumindest einen Filter (23), der an dem Auslass (22) angeordnet ist, und
einen Durchgang der Flüssigkeit durch den Filter ermöglicht, aber einen Durchgang des partikelförmigen Materials durch den Filter verhindert,
wobei der Filter (23) der Flüssigkeit den Durchgang durch den Filter in einer Filterrichtung (x) ermöglicht, wobei der Filter zumindest eine schlitzförmige Öffnung (30) einschließt, welche eine erste Verlängerung (31) und eine zweite Verlängerung (32) aufweist, die im Wesentlichen senkrecht zu der Filterrichtung (x) und der ersten Verlängerung (31) ist,
wobei die zweite Verlängerung (32) wesentlich kleiner als die erste Verlängerung (31) ist, und wobei
die zweite Verlängerung (32) deutlich kürzer als die Länge der schlitzförmigen Öffnung in der Filterrichtung (x) ist.

34. System nach Anspruch 33, bei welchem die Kartusche einen zweiten Filter (24) einschließt, der an dem Einlass (21) angeordnet ist und den Durchgang der Flüssigkeit durch den Filter (24) ermöglicht, aber den Durchgang des partikelförmigen Materials (20) durch den Filter (24) verhindert, wobei der zweite Filter der Flüssigkeit ermöglicht, durch den Filter (24) in einer Filterrichtung (x) zu fließen, **dadurch gekennzeichnet, dass** der zweite Filter zumindest eine schlitzförmige Öffnung einschließt, welche eine erste Verlängerung (31) und eine zweite Verlängerung (32) aufweist, die im Wesentlichen senkrecht zu der Filterrichtung (x) und zu der ersten Verlängerung (31) ist, wobei die zweite Verlängerung (32) wesentlich kürzer als die erste Verlängerung (31) ist.

35. System nach irgendeinem der Ansprüche 33 und 34, **dadurch gekennzeichnet, dass** der Filter die Merkmale von irgendeinem der Ansprüche 2 bis 16 einschließt.

36. System nach irgendeinem der Ansprüche 33 bis 35, bei welchem die Flüssigkeit eine Dialyseflüssigkeit ist.

37. System nach irgendeinem der Ansprüche 33 bis 36, bei welchem das partikelförmige Material Bikarbonat und/oder Natriumchlorid einschließt.

## Revendications

1. Cartouche contenant un matériau en particules (20), dans laquelle la cartouche inclut :
un espace intérieur (10) pour abriter le matériau en particules ;
une entrée (21) agencée pour permettre l'introduction d'un liquide dans l'espace intérieur (10) ;
une sortie (22) agencée pour permettre la décharge de liquide hors de l'espace intérieur (10) ; et
au moins un premier filtre (23) agencé à la sortie (22) et destiné à permettre le passage du liquide à travers le filtre, mais à empêcher le passage du matériau en particules (20) à travers le filtre, dans lequel le filtre permet au liquide de passer à travers le filtre dans une direction de filtration (x),
dans lequel le filtre (23) inclut au moins une ouverture en forme de fente (30), qui présente une première extension (31) et une seconde extension (32) qui est sensiblement perpendiculaire à la direction de filtration (x) et à la première extension, ladite seconde extension (32) étant significativement plus courte que la première extension (31),
**caractérisée en ce que** la seconde extension (32) est significativement plus courte que la longueur de l'ouverture en forme de fente dans la direction de filtration (x).

2. Cartouche selon la revendication 1, dans laquelle la cartouche inclut un second filtre (24) agencé à l'entrée (21) et destiné à permettre le passage du liquide à travers le filtre (24), mais à empêcher le passage du matériau en particules (20) à travers le filtre (24), dans lequel le second filtre permet au liquide de passer à travers le filtre (24) dans une direction de filtration (x), **caractérisée en ce que** le second filtre inclut au moins une ouverture en forme de fente, qui présente une première extension (31) et une seconde extension (32) qui est sensiblement perpendiculaire à la direction de filtration (x) et à la première extension (31), ladite seconde extension (32) étant significativement plus courte que la première extension (31).

3. Cartouche selon l'une quelconque des revendications 1 et 2, **caractérisée en ce que** la seconde extension (32) est égale ou inférieure à 0,12 mm.

4. Cartouche selon l'une quelconque des revendications 1 à 3, **caractérisée en ce que** la seconde extension (32) est égale ou inférieure à 0,10 mm ou 0,08 mm.

5. Cartouche selon l'une quelconque des revendications 1 à 4, **caractérisée en ce que** la seconde extension (32) est égale ou supérieure à 0,02 mm.

6. Cartouche selon l'une quelconque des revendications 1 à 5, **caractérisée en ce que** la seconde extension (32) est égale ou supérieure à 0,04 mm.

7. Cartouche selon l'une quelconque des revendications 1 à 6, **caractérisée en ce que** la seconde extension (32) est approximativement égale à 0,06 mm.

8. Cartouche selon l'une quelconque des revendications 1 à 8, **caractérisée en ce que** le filtre (23, 24) et réalisé en un matériau polymère, qui inclut un matériau parmi le polypropylène et le polycarbonate.

9. Cartouche selon l'une quelconque des revendications 1 à 8, **caractérisée en ce que** la première extension (31) est sensiblement perpendiculaire à la direction de filtration (x).

10. Cartouche selon l'une quelconque des revendications 1 à 9, **caractérisée en ce que** le filtre (23, 24) inclut un élément filtrant (29, 29'), ladite ouverture en forme de fente (30) s'étendant à travers l'élément filtrant.

11. Cartouche selon la revendication 10, **caractérisée en ce que** le filtre (23, 24) inclut une pluralité d'ouvertures en forme de fente (30), qui s'étendent à travers l'élément filtrant (29, 29').

12. Cartouche selon la revendication 11, **caractérisée en ce que** la première extension (31) de chaque ouverture en forme de fente (30) s'étend dans une direction radiale vers un point central de l'élément filtrant (29, 29').

13. Cartouche selon l'une quelconque des revendications 10 à 12, **caractérisée en ce que** l'élément filtrant (29) a la forme d'un disque sensiblement plan.

14. Cartouche selon la revendication 2 et 10, **caractérisée en ce que** l'élément filtrant (29') du second filtre (24) a une forme conique.

15. Cartouche selon l'une quelconque des revendications 10 à 14, **caractérisée en ce que** l'ouverture en forme de fente (30) de l'élément filtrant (29, 29') à une première extrémité est une seconde extrémité, et dans laquelle la seconde extension (32) de l'ouverture en forme de fente augmente depuis une valeur minimum à l'une des extrémités de l'ouverture en forme de fente jusqu'à une valeur maximum à l'autre extrémité de l'ouverture (30).

16. Cartouche selon l'une quelconque des revendications 1 à 15, **caractérisée en ce que** le filtre (23, 24) est réalisé via un processus de moulage par injection

17. Cartouche agencée pour contenir un matériau en particules (20),
dans laquelle la cartouche inclut :
un espace intérieur (10) pour abriter le matériau en particules ;
une entrée (21) agencée pour permettre l'introduction d'un liquide dans l'espace intérieur (10) ;
une sortie (22) agencée pour permettre la décharge du liquide hors de l'espace intérieur (10) ; et
au moins un second filtre (24) agencé à l'entrée (21) et destiné à permettre le passage du liquide à travers le filtre (24) mais à empêcher le passage du matériau en particules (20) à travers le filtre (24), dans laquelle le second filtre permet au liquide de passer à travers le filtre (24) dans une direction de filtration (x),
dans laquelle le second filtre inclut au moins une ouverture en forme de fente, qui présente une première extension (31) et une seconde extension (32) qui est sensiblement perpendiculaire à la direction de filtration (x) et à la première extension (31), ladite seconde extension (32) étant significativement plus courte que la première extension (31),
**caractérisée en ce que** la seconde extension (32) est significativement plus courte que la longueur de l'ouverture en forme de fente dans la direction de filtration (x).

18. Cartouche selon la revendication 17, **caractérisée en ce que** la seconde extension (32) est égale ou inférieure à 0,12 mm.

19. Cartouche selon l'une quelconque des revendications 17 et 18, **caractérisée en ce que** la seconde extension (32) est égale ou inférieure à 0,10 mm ou 0,08 mm.

20. Cartouche selon l'une quelconque des revendications 17 à 19, **caractérisée en ce que** la seconde extension (32) est égale ou supérieure à 0,02 mm.

21. Cartouche selon l'une quelconque des revendications 17 à 20, **caractérisée en ce que** la seconde extension (32) est égale ou supérieure à 0,04 mm.

22. Cartouche selon l'une quelconque des revendications 17 à 21, **caractérisée en ce que** la seconde extension (32) est approximativement égale à 0,06 mm.

23. Cartouche selon l'une quelconque des revendications 17 à 22, **caractérisée en ce que** le filtre (23, 24) est réalisé en un matériau polymère, qui inclut un matériau parmi le polypropylène et le polycarbonate.

24. Cartouche selon l'une quelconque des revendications 17 à 23, **caractérisée en ce que** la première extension (31) est sensiblement perpendiculaire à la direction de filtration (x).

25. Cartouche selon l'une quelconque des revendications 17 à 24, **caractérisée en ce que** le filtre (24) inclut un élément filtrant (29'), ladite ouverture en forme de fente (30) s'étendant à travers l'élément filtrant.

26. Cartouche selon la revendication 25, **caractérisée en ce que** le filtre (23, 24) inclut une pluralité d'ouvertures en forme de fente (30) qui s'étendent à travers l'élément filtrant (29').

27. Cartouche selon la revendication 26, **caractérisée en ce que** la première extension (31) de chaque ouverture en forme de fente (30) s'étend dans une direction radiale vers un point central de l'élément filtrant (29').

28. Cartouche selon l'une quelconque des revendications 25 à 27, **caractérisée en ce que** l'élément filtrant (29) a une forme conique.

29. Cartouche selon l'une quelconque des revendications 25 à 28, **caractérisée en ce que** le filtre (24) inclut une portion de support périphérique connectée à l'élément filtrant (29') et en butée contre une paroi intérieure de la cartouche.

30. Cartouche selon la revendication 29, **caractérisée en ce que** la portion de support périphérique possède une surface périphérique et inclut une pluralité de nervures qui se projettent depuis la surface périphérique et qui viennent buter contre la paroi intérieure de la cartouche, et dans laquelle un intervalle mince est formé entre la surface périphérique et la paroi intérieure, ledit intervalle permettant également un passage pour le liquide.

31. Cartouche selon l'une quelconque des revendications 26 à 30, **caractérisée en ce que** l'ouverture en forme de fente (30) de l'élément filtrant (29') possède une première extrémité et une seconde extrémité, et dans laquelle la seconde extension (32) de l'ouverture en forme de fente diminue de puis une valeur maximum à la première extrémité de l'ouverture en forme de fente jusqu'à une valeur minimum à la seconde extrémité de l'ouverture (30).

32. Cartouche selon l'une quelconque des revendications 17 à 31, **caractérisée en ce que** le filtre (23, 24) est réalisé via un processus de moulage par injection.

33. Système pour préparer une solution liquide pour une procédure médicale, le système incluant :
une cartouche contenant un matériau en particules dans un espace intérieur de la cartouche et incluant une entrée (21) et une sortie (22) ;
un premier conduit à liquide (3) ayant une première extrémité (4) communiquant avec une source (1) de liquide pour extraire le liquide vers le premier conduit à liquide (3), et une seconde extrémité ;
un second conduit à liquide (6) ayant une première extrémité (7) communiquant avec une source (1) de liquide et une seconde extrémité (8) communiquant avec l'entrée de la cartouche (9) pour introduire le liquide dans l'espace intérieur (10) et produire une solution liquide concentrée contenant au moins une partie du matériau en particules dissous dans le liquide ;
un troisième le conduit à liquide (11) communiquant avec la sortie de la cartouche et avec un point de mélange (13) dans le premier conduit à liquide (3) intermédiaire entre ladite première et ladite seconde extrémité (4, 5) pour conduire ladite solution liquide concentrée depuis la cartouche (9) vers ledit premier conduit à liquide pour être mélangée avec le liquide qui est conduit à travers le premier conduit à liquide pour produire ainsi ladite solution liquide dans le premier conduit à liquide pour sa fourniture à la seconde extrémité du premier conduit à liquide ; et
au moins un filtre (23) agencé à la sortie (22) et destiné à permettre le passage du liquide à travers le filtre, mais à empêcher le passage du matériau en particules à travers le filtre, dans lequel le filtre (23) permet au liquide de passer à travers le filtre dans une direction de filtration (x),
dans lequel le filtre inclut au moins une ouverture en forme de fente (30), qui présente une première extension (31) et une seconde extension (32) qui est sensiblement perpendiculaire à la direction de filtration (x) et à la première extension (31), ladite seconde extension (32) étant significativement plus courte que la première extension (31), et
dans lequel la seconde extension (32) est significativement plus courte que la longueur de l'ouverture en forme de fente dans la direction de filtration (x).

34. Système selon la revendication 33, dans lequel la cartouche inclut un second filtre (24) agencé à l'entrée (21) et destiné à permettre le passage du liquide à travers le filtre (24), mais à empêcher un passage du matériau en particules (20) à travers le filtre (24), dans lequel le second filtre permet au liquide de passer à travers le filtre (24) dans une direction de filtration (x), **caractérisé en ce que** le second filtre inclut au moins une ouverture en forme de fente, qui présente une première extension (31) et une seconde extension (32) qui est sensiblement perpendiculaire à la direction de filtration (x) et à la première extension (31), ladite seconde extension (32) étant significativement plus courte que la première extension (31).

35. Système selon l'une quelconque des revendications 33 et 34, **caractérisé en ce que** le filtre inclut les caractéristiques de l'une quelconque des revendications 2 à 16.

36. Système selon l'une quelconque des revendications 33 à 35, dans lequel le liquide est un liquide de dialyse.

37. Système selon l'une quelconque des revendications 33 à 36, dans lequel le matériau en particules inclut du bicarbonate et/ou du chlorure de sodium.
